# EUROPEAN PATENT APPLICATION

(11) **EP 3 427 709 A1**
(43) Date of publication of application: **16.01.2019**
(21) Application number: 17181269.6
(22) Date of filing: 13.07.2017
(51) Int. Cl.: A61F 13/42, G01N 27/04

(54) **WETNESS MONITORING APPARATUS FOR INCONTINENCE SURVEILLANCE**

(71) Applicant: Assistr Digital Health Systems GmbH, 10963 Berlin (DE)
(72) Inventor: GRUDNO, Jens, 10407 Berlin (DE)
(74) Representative: Patent- und Rechtsanwälte Ullrich & Naumann

(57) **Abstract**

The present invention relates to a Wetness monitoring apparatus for incontinence surveillance, comprising
- One or more sensing elements, said sensing elements comprising one or more conductive paths, said conductive paths being printed of one or more conductive co-polymers,
- an absorbent article having an absorbent component, wherein said sensing elements being printed onto one or more components of said absorbent article,
- a readout device being adapted to detect changes in the electrical properties of said sensing elements due to moisture, fluids or the like at least in the vicinity of said sensing elements, and
- a docking component for removably coupling said readout device to said absorbent article, said readout device being electrically connected to said sensing elements when coupled via said docking component.

## Description

The present invention relates to a wetness monitoring apparatus for incontinence surveillance.

The present invention further relates to a method for monitoring wetness of an incontinence event in an absorbent article.

The present invention further relates to a system for monitoring wetness of an incontinence event in an absorbent article.

The present invention even further relates to a non-transitory computer readable medium storing a program causing a computer to execute a method for monitoring wetness of an incontinence event in an absorbent article.

Enuresis or aconuresis is one of the problems a lot of people are faced with. Persons or patients having enuresis or aconuresis need stationary and long-term care which cause high costs for their care. Aconuresis also may cause a lot of secondary diseases: for instance moisture of an incontinence event when being in contact with the human skin over a longer time may lead to perineal skin diseases including bacterial and yeast infections further causing high treatment expenses and major discomfort for the patient.

For treatment of aconuresis incontinence pants are used which should in regular time intervals be checked whether they should be changed. In practice however these intervals are often exceeded. Even when complying with the time plan patients may be subjected to the full time interval length with moisture of their excretions when being incontinent shortly after the checking by the nursery staff. This increases the risk for skin and urinary passage infections.

Permanent control during night further causes discomfort for the patients since their sleep is interrupted. This may in turn significantly increase the likelihood for falls of patients on the following day.

Even further the nursing staff wastes time for unnecessary checks of the incontinence pants, e.g. if no change of the incontinence pant is necessary. Another problem is leakage of the incontinence materials due to a late change. Bed linen, clothing of the patient as well as cleaning of the patient and washing the bed linen are time-consuming and further increase the costs.

In WO 2016/090492 A1 an impedance sensor for detecting and monitoring moisture in absorbent articles are described. An absorbent article includes an impedance sensing element wherein said sensing element includes electrodes. The electrodes may be positioned so as to be capacitivly coupled to an interior region of the absorbent article and to measure an impedance of the absorbent article from the location on the exterior surface of the absorbent article. One of the problems however is that the system and the corresponding impedance sensor are very complicated in their construction as well as in the operation. Another problem is that measurements by the impedance sensor of the impedance are imprecise.

In US 2014/0200538 A1 a liquid sensor is shown having a galvanically energizable power source capable of activating a remotely detectable signal in response to liquid. The liquid sensor includes a plurality of electrodes, a circuit and a transmitter, wherein the electrodes are coupled to generate electric power when in contact with the liquid. The circuit is electrically connected to the electrodes so as to be activated by the electrical power, detect electrical parameters of the electrical power and generate a plurality of data packets indicating a degree of wetness corresponding to the detected electrical parameter. One of the problems however is that the design and consequently the manufacturing process is very complex and complicated. A further problem is that the liquid sensor is expensive, such that a mass production without recycling is not economically worthwhile.

In US 2008/0300559 A1 an absorbent article with a wetness sensor is shown. The wetness sensor comprises an electrical circuit being integrally formed into the absorbent article. The at least one electrical circuit is fabricated from an electrically active material which has been printed onto one or more components of the absorbent article. One of the problems is the complicated integration of a plurality of components directly into the incontinence material during manufacture. A further problem is that the transmission technique used requires a close proximity to the wetness sensor which therefore causes discomfort for the patient as well as is unreliable.

Embodiments of the present invention therefore address the problem of reducing costs for manufacturing, enhanced reliability of detecting wetness in an absorbent article as well as reducing care costs while enhancing comfort for a patient.

In an embodiment the present invention provides a wetness monitoring apparatus for incontinence surveillance, comprising
- One or more sensing elements, said sensing elements comprising one or more conductive paths, said conductive paths being printed of one or more conductive co-polymers,
- an absorbent article having an absorbent component, wherein said sensing elements being printed onto one or more components of said absorbent article,
- a readout device being adapted to detect changes in the electrical properties of said sen sing elementsdue to moisture, fluids or the like at least in the vicinity of said sensing elements, and
- a docking component for removably coupling said readout device to said absorbent article, said readout device being electrically connected to said sensing elements when coupled via said docking component.

In a further embodiment the present invention provides a method for monitoring wetness of an incontinence event in an absorbent article,
comprising the steps of
- Printing one or more sensing elements onto one or more components of said absorbent article, wherein said sensing elements are provided in form of one or more conductive paths, said conductive paths being printed of one or more conductive co-polymers,
- Detecting changes in the electrical properties of said sensing elements by a readout device, due to moisture, fluids or the like at least in the vicinity of said sensing elements,
- Removably coupling said readout device to said absorbent article by a docking component, wherein said readout device being electrically connected to said sensing elements when coupled via said docking component.
- Determining a time point for changing said absorbent article by an evaluation device connected to said readout device.

In a further embodiment the present invention provides a system for monitoring wetness of an incontinence event in an absorbent article, comprising
- one or more sensing elements, said sensing elements comprising one or more conductive paths, said conductive paths being printed of one or more conductive co-polymers,
- an absorbent article having an absorbent component, wherein said sensing elements being printed onto one or more components of said absorbent article,
- a readout device being adapted to detect changes in the electrical properties of said sensing elements due to moisture, fluids or the like at least in the vicinity of said sensing elements, said readout device comprising a sender for transmitting information of detected changes in said electrical properties and
- a docking component for removably coupling said readout device to said absorbent article, said readout device being electrically connected to said sensing elements when coupled via said docking component.
- an evaluation device connected to said readout device, preferably via a wireless connection, said evaluation device comprising a receiver for receiving information from said readout device and being adapted to predict or determine a time point for changing said absorbent article.

In a further embodiment the present invention provides a non-transitory computer readable medium storing a program causing a computer to execute a method for monitoring wetness of an incontinence event in an absorbent article,
comprising the steps of
- printing one or more sensing elements onto one or more components of said absorbent article, wherein said sensing elements are provided in form of one or more conductive paths, said conductive paths being printed of one or more conductive co-polymers,
- detecting changes in the electrical properties of said sensing elements due to moisture, fluids or the like at least in the vicinity of said sensing elements,
- removably coupling said readout device to said absorbent article by a docking component, wherein said readout device being electrically connected to said sensing elements when coupled via said docking component.
- determining a time point for changing said absorbent article by an evaluation device connected to said readout device.

At least one embodiment of the present invention may have at least one of the following advantages:
- enhanced precision in determining moisture, fluids or the like at least in the vicinity of said sensing elements. This enables informing the nursing staff about an optimal time point for changing an incontinence pant of a patient.
- unnecessary changing is avoided, i.e. a change of a incontinence pant too early is avoided saving costs for a new incontinence pant.
- costs associated with leakage of an incontinence pant are avoided.
- reduction of the amount of work for changing an incontinence pant and cleaning a patient.
- low manufacturing costs
- higher reliability
- higher comfort for the nursing staff: Unpleasant cleaning and removing of leakage is reduced in time and in the number of incidents and a patient can be provided with more care time.
- higher comfort for a patient: the system may indicate to a care taker changing an incontinence pant providing dignity of man for the patient. Further the likelihood for illnesses or infects due to a late change of the incontinence pant is avoided.
- Co-polymers provide enhanced stability, solubility, heat resistance, and susceptibility to moisture while offering bio-compatibility to be used in medical products.
- Easy application: The matrix structures of co-polymers offer an easy application for printing onto a substrate, for instance a diaper base in the production process without the need for pretreatment of the substrate. Using other conventional conductive printing inks such as with silver has the drawbacks of being expensive and may give rise to health issues.

The term "vicinity" is to be understood in its broadest sense and refers in particular in the claims, preferably in the specification to an area, region or the like around a sensing element. The form or shape of the area or region is e.g. dependent of the concrete embodiment of the sensing elements in particular their shape and their electric properties. In general the term "vicinity" is meant to be the area around a sensing element in which the electric properties of the sensing element are changed and being detectable due to the presence of moisture, liquid, etc. in this area.

Further features, advantages and further embodiments are described or may become apparent in the following:
Said electrical properties may include resistivity and/or capacity and/or conductance and/or electric potential of said sensing elements. This enables a high flexibility in terms of the electrical properties while enabling high precision in determining moisture, fluids or the like either in direct contact with the sensing elements or in the vicinity of the sensing elements.

Said readout device may be adapted to change from a sleep operating mode to a full operating mode when a change in resistivity is detected. This allows to save energy when no moisture or wetness is detected. Upon waking up due to a change in resistivity, the resistivity and/or another electrical property can be used to determine more precisely the level of change and therefore the amount of moisture, liquid, or the like in the absorbent article. For instance measurement of the resistivity can be used as a wake up signal for the readout device. After being in full operation mode the readout device may perform a capacity measurement for determining level of moisture in the corresponding region of the sensing elements. This enhances the flexibility, saves computational resources and enables a higher precision: For instance an already activated super absorber may avoid in case of the second wetness event that moisture reaches the sensing elements. Therefore a capacity measurement enables higher precision for determining a wetness event due to a change in the electrical properties.

An evaluation device may be connectable to said readout device, preferably via a wireless connection, said readout device comprising a sender for transmitting information of detected changes in said electrical properties to a receiver of said evaluation device. This enables a reliable evaluation. For instance a wetness event can be predicted. The evaluation device may be for instance a cloud computing system with the neural network. Examples for a wireless connection are for example a Bluetooth low energy connection BLE or a sub-GHz LoRa connection.

Said evaluation device may be adapted to predict a leakage of the absorbent article. This enables an optimized change for example in case of incontinence pants; unnecessary checks are avoided and in turn the absorbent article is efficiently used.

Said evaluation device may be adapted to determine the amount of moisture or liquid to be absorbed by the absorbent article and/or the remaining moisture or liquid not being absorbed by said absorbent article. This enables in a very precise way to determine the level of liquid within the absorbent article, and thus the absorbent article can be very efficiently used without the risk of causing skin irritations or the like for patient due to excessive moisture or liquid within the absorbent article.

Said sensing elements may be provided in different regions of said absorbent article, each region having a different leakage probability. This further enhances the precision level for determining whether or not the absorbent article has to be changed or not.

A sensing element may comprise one or more interdigital electrodes. This enables an easy way to provide a cost-effective sensing element.

Said one or more interdigital electrodes may be provided in form of circular, elliptical and/or rectangular spiral and/or intermeshing fingers, said intermeshing fingers each being of t-shaped and u-shaped form and/or each intermeshing finger having a zigzag shaped form. This enhances the flexibility for the interdigital electrodes in terms of their form. Even further the precision for determining moisture or fluids is enhanced, for example in different regions of the absorbent article different forms of the interdigital electrodes may be provided. Also movement of the patient can be taken into account when using different forms of the interdigital electrodes to avoid malfunction and enhance the flexibility: for instance when using intermeshing fingers with a zigzag shaped form stretching of the interdigital electrodes is possible up to a certain level. Therefore reliability is enhanced.

A non-conductive protective layer may be arranged on top of said printed sensing elements. This further enhances the reliability of the wetness monitoring apparatus since short circuits are avoided. The non-conductive protective layer may be printed onto the sensing elements via inkjet printing or via screen printing. An example for a non-conductive and non-harmful material is for example a dye.

Said readout device may be adapted to transmit periodically and/or upon request information about changes in the electric properties of said sensing elements. This enables on the one hand a high reliability to avoid any discomfort for the patient due to a delayed change of the absorbent article and on the other hand transmitting information upon request enhances the reliability such that between scheduled transmissions a further request for information for determining whether the absorbent article has to be changed or not can be provided. Transmitting upon request also saves energy since only when needed/required information is transmitted.

Said readout device may comprise an encrypting component for encrypting information to be transmitted. An encrypting component ensures a high patient privacy and avoids that any unauthorized person can get easy access to patient sensitive information.

Said encrypting component may be adapted to perform encrypting based on a symmetric encryption scheme or a public key/private key encryption scheme. A symmetric encryption scheme enables in an easy way an encryption and saves computational resources in particular of the readout device while a public key/private key encryption scheme enables a high flexibility and high security since for instance a transmission of the key in a secure way is not needed beforehand.

Said readout device may comprise a temporary memory for temporarily storing information to be transmitted and/or an electric power source for power supply of the sensing elements and said temporary memory. This enables to get access to the data of the sensing elements even in case the readout device has no connection to an evaluation device or its electric source has drained. Data loss is therefore avoided.

Said electric power source of said readout device may be chargeable based on movement, temperature difference and/or an electrochemical reaction. This enables an enhanced lifetime of the electric power source of said readout device providing a longer time in operation with the patient.

Said readout device may comprise a location sensor. A location sensor may provide further data for determining a leakage of the absorbent article, for instance if the patient lies on his back the leakage probability is different from the leakage probability when lying on his stomach. Thus precision of a leakage prediction is reduced.

Said co-polymers may comprise polypyrole, polythiophene, polyaniline, poly-3,4-ethylenedioxythiophene and/or polystyrene sulfonic acid. This provides an easy production of the sensing elements based on one or more copolymers. Further when using polythiophene high robustness of the sensing element is provided since polythiophene is highly stable against moisture, temperature and oxygen and is therefore suitable to be used in incontinence products. When a co-polymer is being doped a high conductivity is obtained.

Said readout device may be adapted to perform bidirectional communication with a receiving device to receive indication information for signaling an evaluated result to a person, visually and/or acoustically. This enables informing a patient or the nursery staff for instance about a forthcoming change of an incontinence pant visually, for example by a flashing LED and/or acoustically, for example by playing a tone or the like.

Said docking component may be provided at said readout device. This enables an effective re-use of the readout device since only the sensing elements printed on the absorbent article are dumped in case of a wetted diaper due to an incontinence event.

Said docking component may include a folding mechanism so as to removably coupling said readout device on different sides of the absorbent article. This enables an easy attaching and detaching of the readout device while providing a reliable connection to the absorbent article when being attached.

Said absorbent article may comprise a docking area for coupling said readout device via said docking component, said docking area being located in an upper part of a front side of the absorbent article. This enables an easy attaching and removal of the readout device.

Said docking area of said absorbent article may comprise at least one perforation, preferably three perforations, and said docking component of said readout device may comprise positioning pins for engaging with said perforations of said docking area. This enables an easy attaching of the readout device and a feedback for the person attaching the readout device to the absorbent article.

Said docking area of said absorbent article may comprise at least one connection point, preferably at least five connection points, and said docking component of said read device may comprise at least one connection pin for engaging with said at least one connection point of said docking area. This ensures a reliable electric connection between the readout device and the sensing elements of the absorbent article.

Connection points and connection pins and/or perforations and positioning pins may be symmetrically positioned to each other. This ensures an easy and cost-effective production of the absorbent article and the docking component respectively.

There are several ways how to design and further develop the teaching of the present invention in an advantageous way. To this end, it is to be referred to the patent claims subordinate to the independent claims on the one hand and to the following explanation of preferred examples of embodiments of the invention, illustrated by the drawing on the other hand. In connection with the explanation of the preferred embodiments of the invention by the aid of the drawing, generally preferred embodiments and further developments of the teaching will be explained.

In the drawing
- Fig. 1: shows an overview of a system according to an embodiment of the present invention;
- Fig. 2: shows a cross-section of an absorbent article according to an embodiment of the present invention;
- Fig. 3: shows a sensing element according to an embodiment of the present invention;
- Fig. 4: shows a sensing element according to an embodiment of the present invention;
- Fig. 5a,b: show a graph indicating the resistivity over time for a wetness event according to the embodiment of the present invention (figure 5A) and a further wetness event according to an embodiment of the present invention;
- Fig. 6: shows a wetness monitoring apparatus according to an embodiment of the present invention;
- Fig. 7a,b,c,d: each show a sensing element according an embodiment of the present invention;
- Fig. 8: shows a cross-sectional view of a readout device attached to a diaper according to an embodiment of the present invention;
- Fig. 9: shows a connection component for a docking component according to an embodiment of the present invention; and
- Fig. 10: shows a 3-dimensional view of a diaper and a readout-device

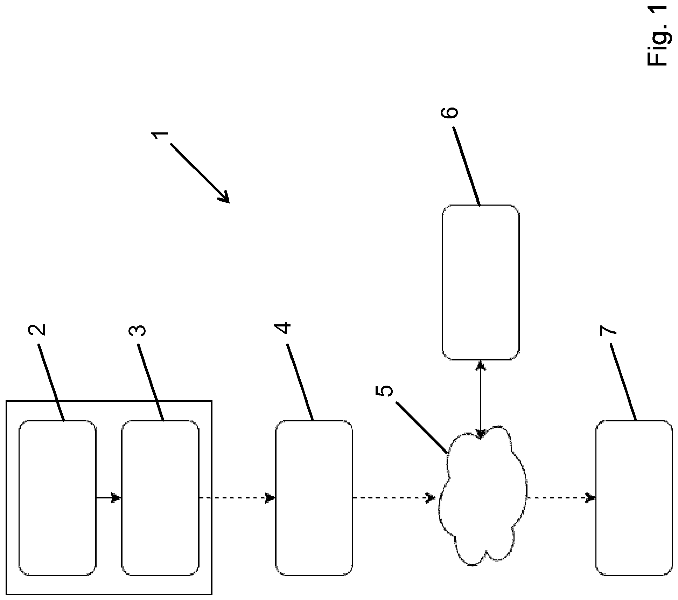

Figure 1 shows an overview of a system according to an embodiment of the present invention.

Figure 1 shows a wetness monitoring apparatus 1 comprising a sensor 2 which is connected to a readout device 3. The readout device 3 is connected to a reception device 4 and the reception device 4 is connected to a server 5 having a web interface 6 for access. The server 5 is being connected to a user equipment 7.

Readout device 3, reception device 4, server 5 and/or user equipment 7 may be connected via a wireless connection using electromagnetic waves for data transmission.

In detail the sensor 2 in connection with the readout device 3 detects moisture, liquid, etc. in the vicinity of the sensor element 3 due to a change in the electric properties of the sensor 3, for example a change in resistivity. The readout device 3 transmits this information via the reception device 4 to a server 5 for evaluation. The server 5 evaluates the received information, i.e. information about the change of resistivity and predicts a possible leakage of the absorbent article of the wetness monitoring apparatus 1. The result is transmitted to said user equipment 7 to inform for example nursing staff that leakage of the absorbent article has occurred or that leakage of the absorbent article is imminent. An application running on the user equipment 7 may indicate to a user when e.g. a diaper of a certain patient has to be changed. The application may also take into account the current position of the care taker for a patient, such that distances, for instance for getting a new diaper are minimized. The server 5 may also provide an adaptable time sheet for changing the absorbent article such that change of the diaper by the nursing staff is optimized. For example the server 5 may adapt the time sheet for charging the diaper of a patient based on recent wetness events of a patient or may provide an estimation for the number of diapers to be used per day, such that always a sufficient number of diapers is available for each patient, etc.. It is also possible to provide a pre-evaluation directly with the readout device 3, when corresponding evaluation mechanisms are provided in an evaluation unit device of said readout device. Further also an evaluation device may be included in said readout device 3 such that transmitting data to a server may not be required or only in case the amount of data to be evaluated exceeds a certain threshold.

Gathering large amounts of electrical properties data - "Big Data" - of wetness events enables not only an optimization of a leakage probability of an incontinence pant, diaper or the like, but also the detection of medically relevant patterns that indicate medical issues. The more data is collected on a personal basis as well as in total, the better certain patterns will be recognized, for instance due to machine learning, neural networks or the like.

The readout device 3 is adapted to measure event strength of a wetness event as well as the variation in time. The readout device 3 encodes the corresponding data and transmits the corresponding data packets to the reception device 4. The readout device 3 may comprise a processor being adapted to perform the procedure, algorithm or the like. The evaluated data can be transmitted via contactless communication to a distant processor, for example the reception device 4 or can be downloaded locally. Nursery staff can be informed and prediction of events can be enhanced as well as electronic electromagnetic documentation of events is enabled.

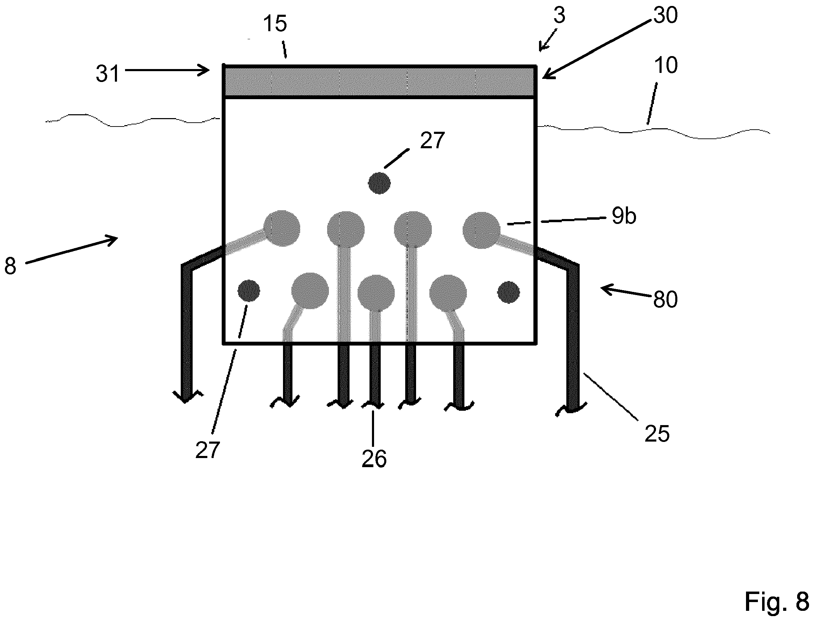

The readout device 3 may be recyclable and is removably attached for example to an incontinence pant by a docking component. The docking component is formed such that the readout device 3 can be attached with high comfort for a patient and such that the connection is robust and the risk for damaging the readout device 3 or a non-desired detachment of the readout device 3 during use is minimized. Such a docking component is for instance shown in Fig. 8-10.

The readout device 3 may as already mentioned above comprises a signal processing entity and a sending entity which transmits continuously or periodically data packets. The readout device 3 further comprises an electric power source, for example a battery or a plurality of batteries, for supplying electrical power to the sensing elements. It is also possible that energy supply for the readout device 3 is provided by a wired connection to an external power source. For instance power supply can also be provided by using movement of the patient, temperature differences or electrochemical reactions with regard to the excretions of the patient. The electric power source may be chargeable: Batteries can be removed, charged and reinserted into the readout device 3 or the readout device 3 can be connected to an external power source for charging the batteries. This can be performed either via a wired connection or via induction.

Unique identifiers may be associated with the readout device 3 which can be included in the generated data packets being sent from the readout device 3 to an evaluation device 5. This enables an association of the generated data packets with the client or patient. Even further the readout device 3 may comprise a surveillance unit in particular for the battery status to avoid an undesired shutdown of the readout device 3.

The readout device 3 may further comprise a signal amplifier, a positional sensor, a docking component and a temporary memory, as well as an encryption module which are described in detail below:
A signal amplifier may be used to amplify signals from the sensing elements which enhances the reliability.

The positional sensor may be used to determine the position and orientation of the patient, for instance if the patient lies, stands or sits. Using this positional data determining a probability for leakage is enhanced and the times needed for changing diapers or the like are further optimized, i.e. reduced.

A temporary memory ensures that in case the wireless connection between the readout device 3 and the reception device 4 or server 5 is broken, data which cannot be transmitted is not lost. The data which cannot be transmitted is stored in the temporary memory until a safe transmission via the reception unit 4 to the server 5 is provided and acknowledged. Then the data may be deleted or overwritten in the temporary memory.

The docking component 8 may be integral part of the readout device 3 and is in particular connectable to a docking area 80 located at the upper edge of the incontinence pant or diaper 10 such that risk of damage and contamination by excretions is minimized. The readout device 3 and the docking component 8 may be formed such that the backside of a back part 30 of the readout device 3 touches the body of a wearer of an incontinence pant 10. This enables with corresponding vital sensors a measurement an evaluation of vital data of a medical patient. When attaching the readout device 3 via the docking component 8 to the docking area 80 of the incontinence pant 10 an electrical connection between the printed conductive sensing elements 2 and the readout device 3 is provided. The electrical contact between the sensing elements 2 and the readout device 3 and the mechanical contact between the docking component 8 and the readout device 3 is provided until a desired detachment of the readout device 3.

The encryption module being part of the readout device 3 can use a symmetric encryption scheme like AES supporting a plot plan of 128 bits and key lengths of 128, 192 and 256 bits. Alternatively or additionally the encryption module may use the public key/secret key encryption scheme. Of course the receiving device may be provided with a corresponding decryption module.

The procedure for the change of an incontinence pant 10 may be as follows:
The readout device 3 is removed from the old incontinence pant 10 and then the readout device 3 is attached to the new incontinence pant 10. During removal as well as during reattachment of the readout device 3 information about attaching and detaching together with a time-code is transmitted to the server 5 via the reception device 4. This enables to measure the time between the occurrence of a wetness event and the change of the incontinence pant 10 enabling the care process to be controlled and optimized. The readout device 3 may signal to a caretaker that it is being attached correctly to the incontinence pant 10 to ensure a connection between the sensing elements 2 and the readout device 3 is present for incontinence surveillance. Signals may be provided optically for example via the light-emitting diode, and in particular vibrations can be used and/or by acoustically, for instance playing a sound when the readout device 3 is correctly attached. The combination of different signals enhances the reliability since the likelihood for recognizing the signals in particular of persons with limited visual and acoustic abilities is increased.

The reception device 4 may also comprise a temporary memory as well as an electric power source similar to the power source described in connection with the readout device 3.

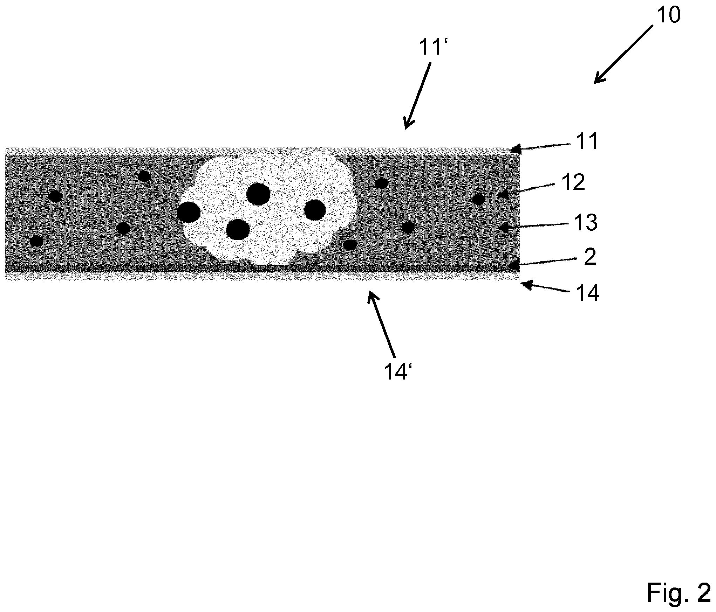

Figure 2 shows a cross-section of an absorbent article according to an embodiment of the present invention.

In figure 2 a cross-section through a diaper 10 is shown. The diaper 10 comprises a plurality of layers which are from the inside 11' to the outside 14' of the diaper 10 as follows: an interior layer 11, a super absorber 12 embedded in a fluff 13, a sensor 2 in form of a sensor strip, and a diaper base 14. The top layer 11 adjacent to a body of a patient is liquid- and moisture-permeable. This top layer 11 is may be an interior non-woven fabric. The diaper base layer 14 is liquid- and moisture-impermeable and avoids leakage of excretions. Between both layers 11, 14 said fluff 13 together with the super absorber 12 being arranged in the fluff 13 is provided. The super absorber 12 may comprise small hygroscopic plastic pellets made of polymers of acrylic acid. These plastic pellets change their state into a gel when liquid is absorbed. Upon entry of liquid into an incontinence pant with an absorber 12 the liquid passes the interior layer 11 and transits through the fluff 13 to the diaper base 14 where it is stopped. Usually the super absorber 12 requires up to 20 seconds to be activated and to bind the liquid. The super absorber 12 may increase its volume up to a factor of 50 when binding the liquid.

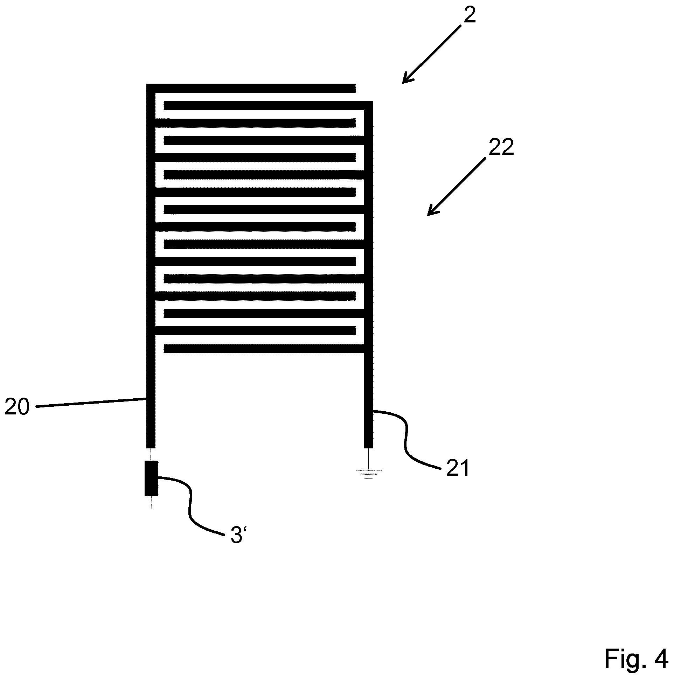

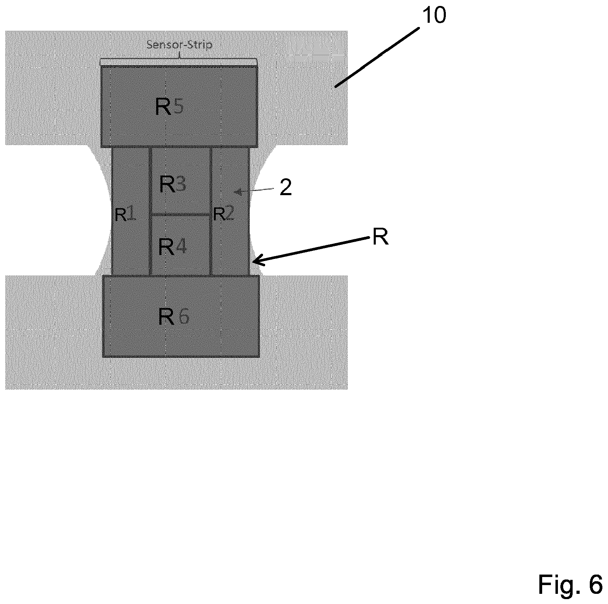

Conductive sensing elements in form a sensor strip 2 are printed on the inside of the diaper base 14, when producing the diaper base 14. The sensing elements 2 may be printed onto material for the diaper base 14 prior to manufacturing the diaper itself. The sensing elements of said sensor strip 2 comprise one or more conductive co-polymers, for instance one co-polymer comprising at least one of: polypyrole, polythiophene, polyaniline, poly-3,4-ethylenedioxythiophene and/or polystyrene sulfonic acid. In particular PEDOT:PSS a combination of poly-3,4-ethylenedioxythiophene and polystyrene sulfonic acid can be used. Of course in general only one co-polymer can be used or also a plurality of co-polymers. Different co-polymers can be used in different regions (see Fig. 6). Also different co-polymers can be used in different areas of the diaper 10, for different electrodes (see Fig. 4) of the sensing elements 2 and/or for different sections of the sensing elements, e.g. different co-polymers for electrodes and for their electric supply lines.

The sensing elements 2 are provided in form of a printable conductive co-polymer being directly printed onto the diaper base 14, said printable conductive co-polymer being polymerizable via infrared and/or ultraviolet light and being bio-compatible. The sensing elements 2 are stable against moisture or liquid for at least 2 hours, preferably for at least 6 hours.

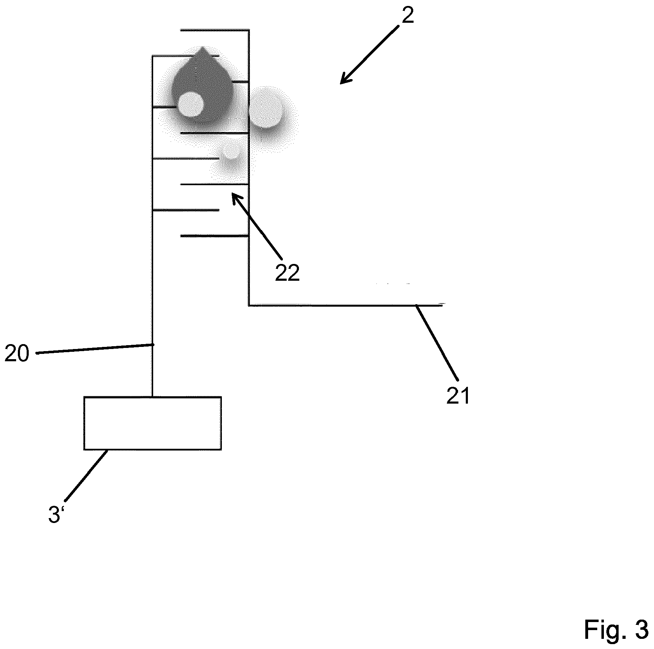

Fig. 3 and Fig. 4 each show a sensing element according to an embodiment of the present invention.

In Fig. 3 a printed sensing element 2 is shown comprising finger-shaped meshing interdigital electrodes 20, 21. The interdigital electrodes 20, 21 - one being connected to a readout-board 3' of the readout-device 3, the other being grounded - are measuring the resistivity against each other, which is lower when a liquid is present. The interdigital electrodes 20, 21 are configured such that they can determine the resistivity and its change between a finger and the two adjacent fingers providing a meshing area 22. The geometry of the interdigital electrodes 20, 21, for instance the form of their meshing area 22, the number of fingers, their lengths and distances can be adjusted such that a desired resistivity and/capacity is provided.

When the interdigital electrodes 20, 21 are subjected to a liquid, for instance urine, the resistivity is lowered due to a higher conductivity of the electrolytes of the urine compared with the diaper base 14 and the air. The difference in resistivity between the neutral resistivity and the resistivity after an excretion event may define the volume of the excretion event which may be additionally based on empirical and/or historical data.

Fig. 4 shows a different sensing element with a finger length greater than the finger length in Fig. 3 and with a smaller distance between the meshing comb-shaped interdigital electrodes 20, 21.

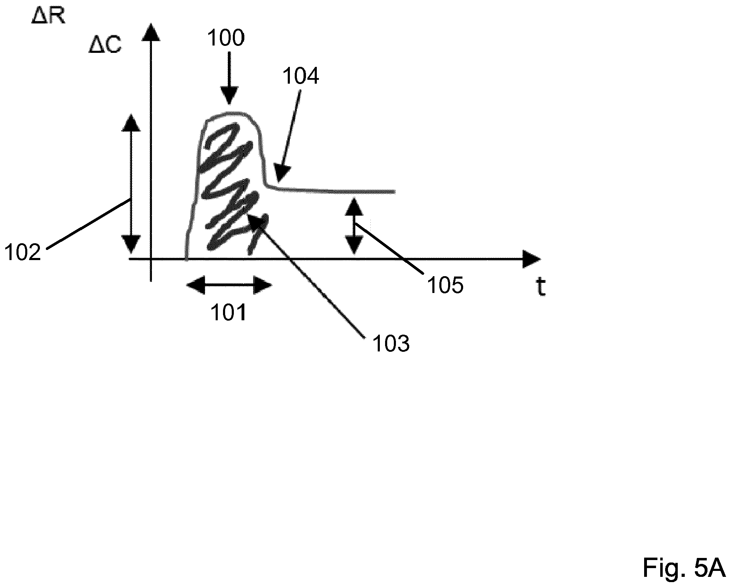

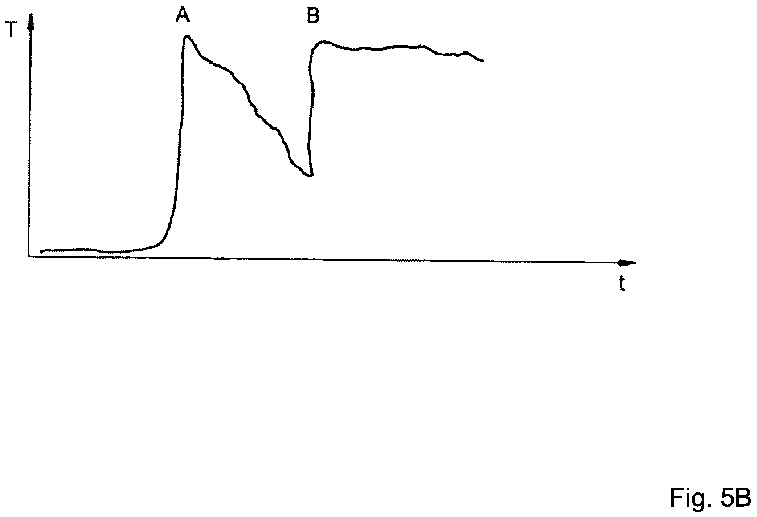

Fig. 5a,b show a graph indicating the resistivity over time for a wetness event according to the embodiment of the present invention (fig. 5a) and occurrence of two subsequent wetness events according to an embodiment of the present invention (fig. 5b).

In Fig. 5a a change in resistivity and/or capacity of a sensing element 2 is shown. In detail in Fig. 5a shows the change in resistivity and/or capacity at a sensing element 2 over time t when a wetness event 100, for instance a patient wets his diaper 10, occurs. The intensity 102 of the wetness event 100 is indicated by a change in the absolute value of the resistivity and this is an indication for the amount of liquid, e.g. urine. The time duration 101 of the wetness event 100 is associated with the time a super absorber 12 needs for being activated and binding the liquid. The binding of the liquid by the super absorber 12 forces the liquid to the middle of the super-absorber 12 off the sensing elements 2. This causes a rise in the resistivity and a decrease 104 of the curve in Fig. 5a. The remaining moisture due to regions of the super-absorber 12 not being able to bind the liquid is a remaining difference in the resistivity, indicated by reference sign 105.

Fig. 5b now shows a second wetness event B after occurrence of a first wetness event A, said wetness event A being similar to the wetness event 100 of Fig. 5a. After occurrence of the wetness event B the difference in resistivity measured by a sensing element 2 not being subjected to wetness or moisture is maintained, i.e. the super-absorber 12 cannot bind further liquid. This means the binding capacity of the super absorber 12 is at its maximum; the liquid can therefore move within the absorbent layer and cause skin irritations or the like for a patient and the likelihood for leakage is high. Wetness event B may also associated with a defecating event, where the defecating material cannot be absorbed by the super absorber 12.

For determining the position of the moisture - as mentioned above - a resistivity and/or a capacity measurement is used. The level of local conductance or the time difference in the local resistivity enables an enhanced precision when determining the volume of the liquid. The changing resistivity occurs depending on the amount of liquid between the meshing interdigital electrodes: resistivity is lower with increasing moisture/liquid.

Figure 6 shows a wetness monitoring apparatus according to an embodiment of the present invention.

In detail in figure 6 a diaper 10 in its unfolded state is shown. The sensing elements 2 are located in different zones or regions, in figure 6 six regions R1-R6 are shown. The readout device 3 is able to associate received measured data from the sensing elements to the corresponding regions and to analyze the data. The sensing element(s) 2 can determine the location or position of the liquid volume precisely since different regions located in different areas of the diaper/incontinence pant 10, are associated with different leakage probability. For example in the regions R1 and R2 leakage probability is statistically higher than in regions R5 and R6. The regions are R1-R6 will be weight differently when being evaluated together to determine the leakage probability very precisely and to determine, to adapt or to update schedule for changing the diaper 10.

The evaluation of the data may be performed based on certain criteria for example the level of comfort for the patient, a maximum time between subsequent changes, cost minimization, etc.

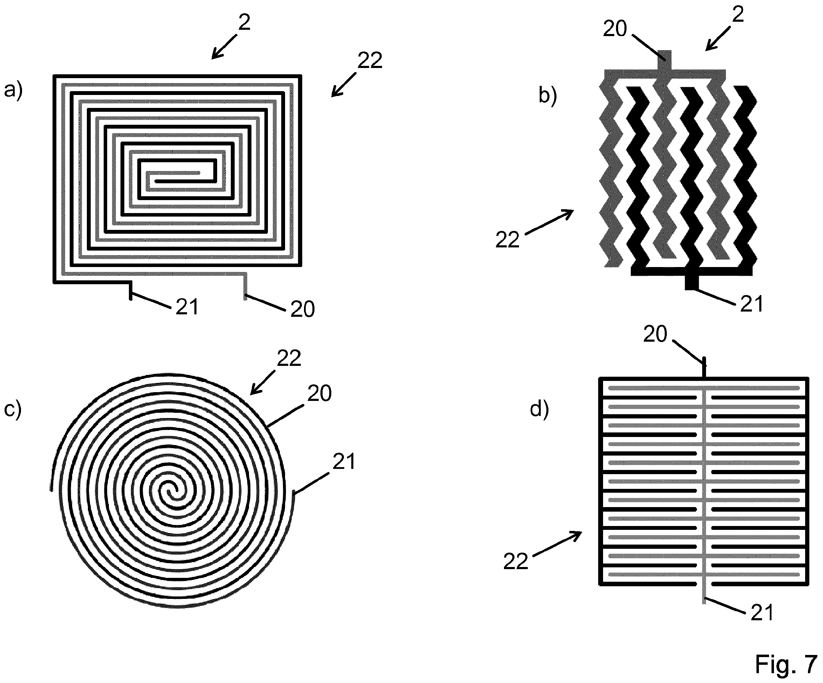

Figures 7a-7d each show a sensing element according an embodiment of the present invention.

Figure 7a shows a rectangular spiral arrangement of the two interdigital electrodes 20, 21, whereas figure 7c shows a corresponding circular spiral arrangement of the two interdigital electrodes 20, 21. Figure 7b shows a zigzag structure of meshing fingers, each of the fingers having a zigzag form wherein adjacent fingers having in general the same zigzag form. Figure 7d shows tree-like electrodes 21 intermeshing with an enveloping second electrode 22 having a plurality of U-shaped fingers surrounding at least in part the fingers of the tree structure being form of multiple T-shaped fingers.

Sensing elements according to embodiments of the present invention may be printed with ink enabling line widths and distances between the interdigital electrodes of 100 µm or less. The sensing elements may comprise conductive copolymers being a synthetic material with electrical conductivity where their conjugated double bonds enable the conductance with which in the doped state the free movement of charge carriers is enabled.

For instance sensing elements may comprise polypyrole, polythiophene, polyaniline, poly-3,4-ethylenedioxythiophene and/or polystyrene sulfonic acid. Printing may be performed by an ink-jet printing method enabling an easy printing on the flexible substrates like PE, synthetic polymer foils or coated paper. Additionally or alternatively screen printing, offset printing, flexographic printing and/or gravure printing may be used.

Fig. 8 shows a cross-sectional view of a readout device attached to a diaper according to an embodiment of the present invention.

In Fig. 8 a diaper 10 with a readout device 3 is shown attached to a diaper via a docking component 8. The docking component 8 is integrated into the diaper 10 and comprises a plurality of positioning perforations 27, in Fig. 8 three positioning perforations being arranged in triangular manner to each other. These positioning perforations correspond to positioning pins 28 of the readout device 3 which interact with the positioning perforations 27 of the diaper 10 in the attached state of the readout device 3 to the diaper 10 such that here they extent through the positioning perforations 27 and can be removably locked in their positions. The docking component 8 further comprises a plurality of connection pins 9b which electrically connect to corresponding connection points 9a of the diaper 10. The connections points 9a of the diaper 10 are in turn electrically connected to sensor lanes 25 and a grounded lane 26 of the sensing elements.

The readout device 3 in Fig. 8 comprises - as already mentioned - two components, a front part 29 and a back part 30 which can be rotated to each other along a common axis 31 via a corresponding clip mechanics 15. Front part 29 and back part 30 of the readout device 3 can be removably locked or engaged such that the positioning pins 28 of the readout device 3 extent through the positioning perforations 27 of the diaper 10 and such that an electric connection between the connection points 9a of the diaper 10 and the connection pins 9b of the readout device 3.

In the embodiments of Fig. 8-10 the connection pins 9b and the positioning pins 28 of the readout device 3 extent from the front side of the back part 30 of the readout device 3. It is also possible to provide the connection pins 9b and/or the positioning pins 28 on the back side of the front part 29 or on different sides, for instance connection pins 9b on the back side of front part 29 and positioning pins 28 on the front side of back part 30 or vice versa or the like. The docking area 80 comprises then a front side and back side with the connection points 9a and positioning perforations 27 being arranged correspondingly to the embodiment of the readout device 3.

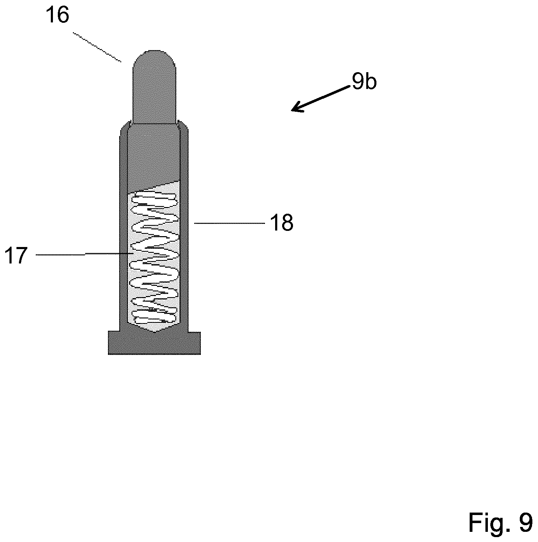

Fig. 9 shows a connection component for a docking component according to an embodiment of the present invention.

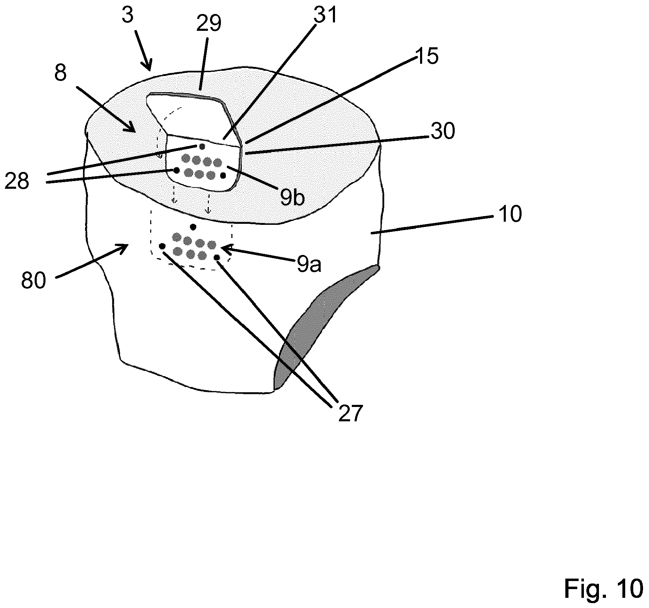

In Fig. 9 in detail a spring-loaded pin 9b is shown comprising a body or housing 18 in which a spring 17 is positioned. At the upper end of the housing a pin 16 is provided interacting with the spring 17. When a force is applied on the pin 16 in direction of the spring 17, the spring 17 is compressed and the pin 16 is further moved into the housing 18. If the force is not present anymore the spring 17 decompresses and the pin 16 is moved to its former position. Fig. 10 shows a 3-dimensional view of a diaper and a readout-device.

In Fig. 10 in detail shows a diaper 10 comprising on the upper inside connection points 9a for electrically connecting a readout device 3 to sensing elements inside the diaper 10 as well as positioning perforations 27 for positioning a readout device 3 correctly. Further in Fig. 10 a readout device 3 is shown comprising a front part 29 and a back part 30, mechanically connected via a clip mechanic 15, such that front part 29 and back part 30 can be folded, i.e. front part 29 and back part 30 are facing directly to each other and such that the readout device 3 is correctly attached to the docking component 8 of the diaper 10: connection pins 9b are electrically connected to the corresponding connection points 9a of the diaper 10 and positioning pins 28 extending from the back part 30 to the front part 29 extent through the positioning perforations 27 of the diaper 10.

The contact closure between the sensing strips/sensing elements of the diaper 10 with their possible conductive paths and the readout device 3 can also be established for instance through variations of plug and socket connections, interlocking connectors, magnetic connectors, spring-loaded pins, or clamps with or without one or more springs 17. The correct mounting point for the readout device 3 can be made visible optically and/or through form closure /mechanical restraint, for example through perforation at the diaper or fixtures that are applied to the diaper during or after the production process.

Fig. 10 shows a diaper 10 with three perforations in the upper part on the front part 29 of the diaper 10. Further seven ends of sensing elements 2 are also depicted, while the end points - connection points 9a - can be larger in diameter than the printed sensing strips to include a margin of error when docking the readout device 3, for instance a margin of at least 10% of the diameter of the connection pin or the like, preferably at least 30%. One connection point 9a is connected to a grounded lane 26. The readout device 3 is mounted to the diaper 10 from the top, while the back part 30 of the readout device 3 is in contact with the connection points 9a of the sensing elements 2 and the perforations 27 are, in this example, entered by three nipples from the inside of the diaper 10 to the outside. The part 29 of the readout device 3 that is on the outside of the diaper 10 is then folded down to close it. Once closed, the connection points 9a of the sensing elements 2 are in contact here with seven spring-loaded pins 9b from the readout device 3. The readout device 3 has a snap mechanism to avoid an unintentional opening. Once the readout device 3 is attached to the diaper 10, a time stamp may be sent to a server 5 for reference. Also, the person attaching the readout device 3 may receive a feedback if the readout device 3 was mounted correctly or not.

In summary the present invention, in particular their embodiments enable or provide a system for determining moisture comprising sensing elements being of a conductive co-polymer enabling a determination of a change in moisture due to a change in the electrical properties, e.g. resistivity. The generated data can be transmitted for remote control from a readout device to third party devices, for example a reception device in connection with a server infrastructure and user equipment like smart phones or the like.

Embodiments of the present invention may provide an optimized schedule for changing incontinence products, devices or the like using procedures based on locally measured differences in the electrical properties and which can be transformed into behavior guidelines for nursery staff. Even further embodiments of the present invention provide printed liquid detection sensors with enhanced sensing properties, enable low costs in mass production compared with conventional methods and systems and an enhanced incontinence care management. In particular a predicted time point for a change of incontinence devices or the like is provided e.g. due to an evaluation by a server infrastructure enabling to detect anomalies of each patient much earlier than the nursery staff is able to. Even further control of the nursery staff and their working guidelines and processes can be provided.

Many modifications and other embodiments of the invention set forth herein will come to mind to the one skilled in the art to which the invention pertains having the benefit of the teachings presented in the foregoing description and the associated drawings. Therefore, it is to be understood that the invention is not to be limited to the specific embodiments disclosed and that modifications and other embodiments are intended to be included within the scope of the appended claims. Although specific terms are employed herein, they are used in a generic and descriptive sense only and not for purposes of limitation.

### List of reference signs

- 1: wetness monitoring apparatus
- 2: sensing strip/sensing element
- 3: readout device
- 3': readout board
- 4: reception device
- 5: server
- 6: web interface
- 7: user equipment
- 8: docking component
- 9a: connection point diaper
- 9b: connection point docking component
- 10: diaper
- 11: inner layer
- 11': inner surface
- 12: absorber
- 13: fluff
- 14: diaper base
- 14': outer surface
- 15: clip mechanics
- 16: pin
- 17: spring
- 18: body/housing
- 20, 21: Electrode
- 22: meshing area
- 25: sensor lane
- 26: grounded lane
- 27: positioning perforation
- 28: positioning pin
- 29: front part
- 30: back part
- 31: axis
- 80: docking area
- 100: wetness event
- 101: time duration
- 102: intensity
- 103: volume
- 104: decrease
- 105: remaining resistivity/capacity
- A, B: wetness event
- R1, R2, R3, R4, R5, R6: region

## Claims

1. Wetness monitoring apparatus for incontinence surveillance, comprising
- One or more sensing elements (2), said sensing elements (2) comprising one or more conductive paths (25, 26), said conductive paths (25, 26) being printed of one or more conductive co-polymers,
- an absorbent article (10) having an absorbent component (12), wherein said sensing elements (2) being printed onto one or more components (11, 12, 13, 14) of said absorbent article (10),
- a readout device (3) being adapted to detect changes in the electrical properties of said sensing elements (2) due to moisture, fluids or the like at least in the vicinity of said sensing elements (2), and
- a docking component (8) for removably coupling said readout device (3) to said absorbent article (10), said readout device (3) being electrically connected to said sensing elements (2) when coupled via said docking component (8),
wherein said electrical properties may include resistivity and/or capacity and/or conductance and/or electric potential of said sensing elements (2) and wherein said readout device (3) is adapted to change from a sleep operating mode to a full operating mode when a change in resistivity is detected.

2. Wetness monitoring apparatus according to claim 1, wherein an evaluation device (5) is connectable to said readout device (3), preferably via a wireless connection, said readout device (3) comprising a sender for transmitting information of detected changes in said electrical properties to a receiver of said evaluation device (5), preferably wherein said evaluation device (5) is adapted to predict a leakage of the absorbent article (10).

3. Wetness monitoring apparatus according to one of the claims 1-2, wherein said evaluation device (5) is adapted to determine the amount of moisture or liquid to be absorbed by the absorbent article (10) and/or the remaining moisture or liquid not being absorbed by said absorbent article (10).

4. Wetness monitoring apparatus according to one of the claims 1-3, wherein said sensing elements (2) are provided in different regions (R1, R2, R3, R4, R5, R6) of said absorbent article (10), each region (R1, R2, R3, R4, R5, R6) having a different leakage probability.

5. Wetness monitoring apparatus according to one of the claims 1-4, wherein a sensing element (2) comprises one or more interdigital electrodes (20, 21), preferably wherein said one or more interdigital electrodes (20, 21) are provided in form of circular, elliptical and/or rectangular spiral and/or intermeshing fingers (22), said intermeshing fingers (22) each being of t-shaped and u-shaped form and/or each intermeshing finger having a zigzag shaped form.

6. Wetness monitoring apparatus according to one of the claims 1-5, wherein a non-conductive protective layer is arranged on top of said printed sensing elements (2) and/or wherein said co-polymers comprise polypyrole, polythiophene, polyaniline, poly-3,4-ethylenedioxythiophene and/or polystyrene sulfonic acid.

7. Wetness monitoring apparatus according to one of the claims 1-6, wherein said readout device (3) is adapted to transmit periodically and/or upon request information about changes in the electric properties of said sensing elements (2) and/or wherein said readout device (3) comprises an encrypting component for encrypting information to be transmitted, preferably wherein said encrypting component is adapted to perform encrypting based on a symmetric encryption scheme or a public key/private key encryption scheme.

8. Wetness monitoring apparatus according to one of the claims 1-7, wherein said readout device (3) comprises a temporary memory for temporarily storing information to be transmitted and/or an electric power source for power supply of the sensing elements (2) and said temporary memory, preferably wherein said electric power source of said readout device (3) is chargeable based on movement, temperature difference and/or an electrochemical reaction and/or a location sensor.

9. Wetness monitoring apparatus according to one of the claims 1-8, wherein said readout device (3) is adapted to perform bidirectional communication with a receiving device (4, 5) to receive indication information for signaling an evaluated result to a person, visually and/or acoustically.

10. Wetness monitoring apparatus according to one of the claims 1-9, wherein said docking component (8) is provided at said readout device (3) and/or wherein said docking component (8) includes a folding mechanism (15, 31) so as to removably coupling said readout device (3) on different sides of the absorbent article (10).

11. Wetness monitoring apparatus according to claim 10, wherein said absorbent article (10) comprises a docking area (80) for coupling said readout device (3) via said docking component (8), preferably wherein said docking area (80) being located in an upper part of a front side of the absorbent article (10),
preferably wherein said docking area (80) of said absorbent article (10) comprises at least one perforation (27), preferably three perforations, and said docking component (8) of said readout device (3) comprises corresponding positioning pins (28) for engaging with said perforations (27) of said docking area (80) and/or wherein said docking area (80) of said absorbent article (10) comprises at least one connection point (9a), preferably at least five connection points (9a), and said docking component (8) of said readout device (3) comprises at least one connection pin (9b) for engaging with said at least one connection point (9a) of said docking area (80).

12. Wetness monitoring apparatus according to claim 11, wherein connection points (9a) and connection pins (9b) and/or perforations (27) and positioning pins (18) are symmetrically positioned to each other.

13. Method for monitoring wetness of an incontinence event in an absorbent article (10), comprising the steps of
- Printing one or more sensing elements (2) onto one or more components (11, 12, 13, 14) of said absorbent article (10), wherein said sensing elements (2) are provided in form of one or more conductive paths (25, 26), said conductive paths (25, 26) being printed of one or more conductive co-polymers,
- Detecting changes in the electrical properties of said sensing elements (2) by a readout device (3), due to moisture, fluids or the like at least in the vicinity of said sensing elements (2),
- Removably coupling said readout device (3) to said absorbent article (10) by a docking component (8), wherein said readout device (3) being electrically connected to said sensing elements (2) when coupled via said docking component (8).
- Determining a time point for changing said absorbent article by an evaluation device (5) connected to said readout device (3).

14. System for monitoring wetness of an incontinence event in an absorbent article (10),
comprising
- one or more sensing elements (2), said sensing elements (2) comprising one or more conductive paths (25, 26), said conductive paths (25, 26) being printed of one or more conductive co-polymers,
- an absorbent article (10) having an absorbent component (12), wherein said sensing elements (2) being printed onto one or more components (11, 12, 13, 14) of said absorbent article (10),
- a readout device (3) being adapted to detect changes in the electrical properties of said sensing elements (2) due to moisture, fluids or the like at least in the vicinity of said sensing elements (2), said readout device comprising a sender for transmitting information of detected changes in said electrical properties and
- a docking component (8) for removably coupling said readout device (3) to said absorbent article (10), said readout device (3) being electrically connected to said sensing elements (2) when coupled via said docking component (8).
- an evaluation device (5) connected to said readout device (3), preferably via a wireless connection, said evaluation device (5) comprising a receiver for receiving information from said readout device (3) and being adapted to predict or determine a time point for changing said absorbent article (10).

15. A non-transitory computer readable medium storing a program causing a computer to execute a method for monitoring wetness of an incontinence event in an absorbent article (10),
comprising the steps of
- printing one or more sensing elements (2) onto one or more components (11, 12, 13, 14) of said absorbent article (10), wherein said sensing elements (2) are provided in form of one or more conductive paths (25, 26), said conductive paths (25, 26) being printed of one or more conductive co-polymers,
- detecting changes in the electrical properties of said sensing elements (2)due to moisture, fluids or the like at least in the vicinity of said sensing elements (2),
- removably coupling said readout device (3) to said absorbent article (10) by a docking component (8), wherein said readout device (3) being electrically connected to said sensing elements (2) when coupled via said docking component (8).
- determining a time point for changing said absorbent article by an evaluation device (5) connected to said readout device (3).
